(19) Europäisches Patentamt
European Patent Office
Office européen des brevets

(11) Publication number : **0 516 529 A2**

# (12) EUROPEAN PATENT APPLICATION

(21) Application number : **92401437.6**

(22) Date of filing : **26.05.92**

(51) Int. Cl.$^5$ : **G01N 33/68,** // G01N33/569, G01N33/576

(30) Priority : **28.05.91 JP 123574/91**

(43) Date of publication of application : **02.12.92 Bulletin 92/49**

(84) Designated Contracting States : **AT BE CH DE DK ES FR GB GR IT LI LU MC NL PT SE**

(71) Applicant : **DAIICHI PURE CHEMICALS CO. LTD.**
**13-5, Nihonbashi 3-chome**
**Chuo-ku Tokyo (JP)**

(72) Inventor : **Xu, Jing-Zhi**
**25 Drake Crescent**
**St. John's Nfld., A1A 3L2 (CA)**
Inventor : **Matsuo, Masanao**
**Takitaso-C, 600, futako**
**Takatsu-Ku, Kawasaki-shi, Kanagawa (JP)**
Inventor : **Kamiyama**
**12-4, Minamisuma 3-chome**
**Koto-ky, Tokyo (JP)**
Inventor : **Teramura, Yasuo**
**20-9-306, Nishikasai 3-Chome**
**Edogawa-ku, Tokyo (JP)**
Inventor : **Sakai, Yasuo**
**5-1-505, Harayama 3-Chome**
**Inzai-machi, Inba-gun, Chiba (JP)**

(74) Representative : **Orès, Bernard et al**
**Cabinet ORES 6, Avenue de Messine**
**F-75008 Paris (FR)**

## (54) Assay of specific antibody.

(57) Disclosed herein is an assay of a specific antibody to an antigen in a particular immunoglobulin class and/or subclass by an immunoassay wherein an immunological agglutination making use of antigen-sensitized insoluble carrier particles and a second antibody specific to the particular immunoglobulin class and/or subclass is used. A trivalent or higher polyvalent antibody specific to the particular immunoglobulin class and/or subclass is used as the second antibody.

FIG. 1

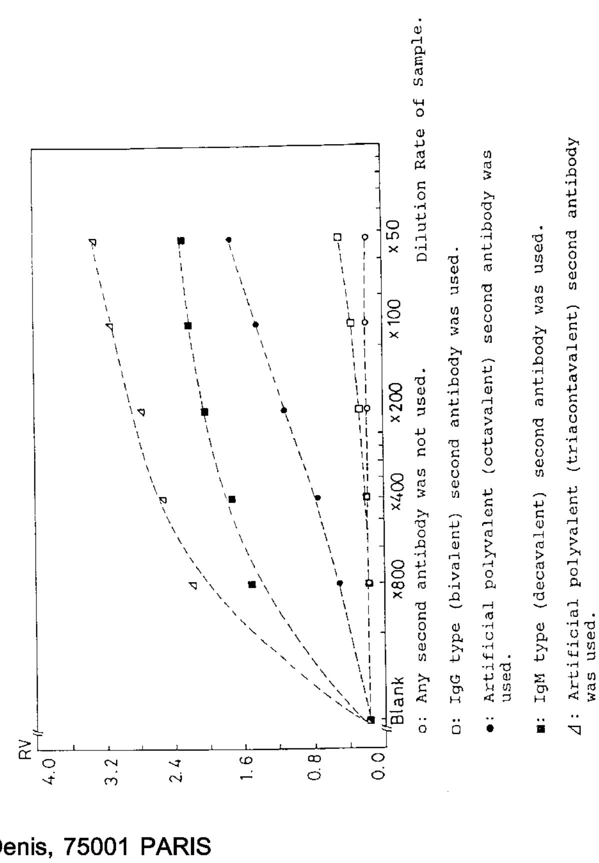


EP 0 516 529 A2

## BACKGROUND OF THE INVENTION

### i) Field of the Invention:

This invention relates to an assay of a specific antibody making use of an immunological agglutination, and more specifically to an assay of a specific antibody, which permits the selective detection of the specific antibody intended to assay with high sensitivity by amplifying only the agglutination caused by such a specific antibody.

### ii) Description of the Background Art:

Specific antibodies belonging to various classes (for example, IgG, IgA, IgM, IgE, IgD) and subclasses coexist in immunoglobulins in serum. It is extremely important for the diagnosis of diseases to assay only a specific antibody of a particular class and/or subclass (hereinafter referred to as the "particular class" simply). For example, the assay of a specific IgG antibody is useful to determine whether the specimen has been sensitized by its antigen, bacterium or virus in the past. Its antibody titer, however, begins rising only after a sufficient period of time has elapsed. It is possible to find the sensitization of about several days to 2 weeks ago by assaying a specific IgM at this time. In various kinds of allergic diseases, it is very useful to assay the respective specific antibodies to their allergens. Information obtainable from the diagnosis varies according to the antibodies of individual classes. Namely, a specific IgE antibody is useful to search a causative allergen of immediate-type allergy. A specific IgG antibody is useful to search a causative allergen of delayed-type allergy and grasp the degree of an advanced disease. Besides, it is said that a specific IgA antibody participates in the prophylaxis of allergic crisis.

As described in Ito, Koji et al., Allergy, 33, 158(1984), such specific antibodies of different classes have heretofore been assayed by the enzyme immunoassay (EIA) making use of enzyme-labelled second antibodies specific to the individual classes. However, the assay in accordance with this EIA involves a drawback that the assaying operation include many steps and is hence complicated, and it also takes a lot of time.

On the other hand, immunological agglutination making use of insoluble carrier particles is widely used as a method simple in process, short in assaying time and high in sensitivity. There are many sorts of detection methods such as turbidimetry [Dezelic, N. et al., Croat. Chem. Acta., 42, 457(1970)], nephelometry [Cohen, R.J. et al., Immuno-chemistry, 13, 963(1976)], DALIA method [Sakai, Y. et al, Chem. Pharm. Bull., 37, 3010(1989)] and PACIA method [Cambiaso, C.L. et al., J. Immunol. Methods, 18, 33(1977)].

In order to assay only a specific antibody included in a particular class in serum by using the immunological agglutination making use of these insoluble carrier particles, there are several problems. For example, since specific antibodies of various classes to antigens exist in serum, it is impossible even from the theoretical viewpoint to directly assay only the specific antibody included in the particular class by using the immunological agglutination making use of insoluble carrier particles sensitized with an antigen. As a method of solving this problem, it has been proposed to remove antibodies of unnecessary classes in advance. This method involves a problem in that it requires a complicated pretreatment operation. As described above, it is extremely useful from the viewpoint of diagnosis to assay a specific antibody of a particular class without conducting such a pretreatment and free from the interference of antibodies of other classes.

By the way, the concentration of a specific antibody in a particular class existing in serum is extremely low compared with the total concentration of antibodies in the particular class. Therefore, the specific antibody may be difficult to assay in many cases due to its insufficient sensitivity upon the assay even if the assay can be conducted free from such interference of the antibodies of other classes as described above.

## SUMMARY OF THE INVENTION

Therefore, an object of this invention is to provide a method of assaying only a specific antibody to an antigen in a particular immunoglobulin class intended to assay with high sensitivity by using an immunological agglutination making use of an antigen-sensitized insoluble carrier.

The present inventors have carried out an extensive investigation with a view toward achieving the above object. As a result, it has been found that when a trivalent or higher polyvalent antibody, i.e., an antibody having three or more binding sites to a specific antibody intended to assay is used as a second antibody, the agglutination by the specific antibody is amplified, so that the detection sensitivity of the specific antibody intended to assay is sharply improved, leading to completion of this invention.

In an aspect of this invention, there is thus provided an assay of a specific antibody to an antigen in a particular immunoglobulin class by an immunoassay wherein an immunological agglutination making use of antigen-sensitized insoluble carrier particles and a second antibody specific to the particular immunoglobulin class is used, which comprises using a trivalent or higher polyvalent antibody specific to the particular immunoglobulin class as the second antibody.

## BRIEF DESCRIPTION OF THE DRAWINGS

The above and other objects, features and advantages of the present invention will become apparent from the following description and the appended claims, taken in conjunction with the accompanying drawings, in which:

FIG. 1 illustrates a dilution reaction curve of a mite-specific IgG;

FIG. 2 illustrates a dilution reaction curve of a mite-specific IgA;

FIG. 3 illustrates a dilution reaction curve of a cedar-specific IgG;

FIG. 4 illustrates a dilution reaction curve of a rice-specific IgG;

FIG. 5 illustrates a dilution reaction curve of a rice-specific IgA;

FIG. 6 illustrates a dilution reaction curve of an HBs-specific IgG;

FIG. 7 illustrates a dilution reaction curve of a candida-specific IgG;

FIG. 8 illustrates a dilution reaction curve of a candida-specific IgM; and

FIG. 9 illustrates an inhibition reaction curve (as to the mite-specific IgG) by a mite antigen.

## DETAILED DESCRIPTION OF THE INVENTION AND PREFERRED EMBODIMENTS

As human fluid samples used in this invention, may be mentioned samples containing a specific antibody of a particular class intended to assay (hereinafter referred to as "the specific antibody to be assayed"), for example, plasma, serum, snivel, saliva, tear, cerebrospinal fluid, etc. In these human fluid samples, specific antibodies (hereinafter referred to as "competitive antibodies") other than that of the particular class intended to assay exist together with the specific antibody to be assayed. Therefore, the sample is preferably diluted before use (hereinafter referred to as "the diluted specimen") to such a concentration level that the antigen-sensitized insoluble carrier particles are not aggregated solely by the specific antibody to be assayed and the competitive antibodies in the sample.

As the insoluble carrier particles, there can be used high-molecular weight carrier particles of such a type as polyamide, polyvinylidene chloride, polyvinyl chloride, acrylics, polyvinyl acetate, polystyrene, polypropylene, polyepoxy, urethanes, polyethylene, agarose, chitosan or pullulane. These insoluble carrier particles preferably have active groups such as carboxyl group, sulfonic group or amino group on their surfaces in a predetermined proportion. Those having a particle size in a range of, preferably, 0.1-3.0 microns, particularly, 0.1-1.2 microns may be used.

The antigen by which the insoluble carrier particles are sensitized is an antigen corresponding to the specific antibody to be assayed. As specific examples thereof, may be mentioned various allergens such as mite, cedar pollen, rice, candida and egg white; viral antigens such as hepatitis B virus and adenovirus; bacterial antigens such as Chlamydia and Treponema pallidum; fungal antigens such as Aspergillus; parasitic antigens such as Toxoplasma and Rickettsia; and the like. These antigens may be used as they are or in the form of a fraction partially purified by extraction or the like in accordance with the conventional method.

It is only necessary to follow a method known per se in the art in order to sensitize the insoluble carrier particles with the antigen. This sensitization is effected, for example, by a physical adsorption process in which a solution with an antigen dissolved in a buffer is added to a suspension of the insoluble carrier particles in a buffer, the resultant mixture is left over for from 30 minutes to 24 hours and the antigen unsensitized is then removed by means such as centrifugation. Alternatively, the sensitization is performed by a chemical covalent bonding process in which an antigen is sensitized on the insoluble carrier particles by using a known bifunctional crosslinking reagent or binder. The concentration of the antigen upon the sensitization is properly about 0.1-2 wt.% in general. Of course, it may however be changed suitably according to various conditions such as the kind and properties of the antigen, and the material of the insoluble carrier particles and nature of active groups existing on their surfaces. The antigen-sensitized insoluble carrier particles (hereinafter abbreviated as "the insoluble antigenic reagent") thus obtained may also be preserved by treating them with proteins free of any antigenicity, various kinds of surfactants and the like and then suspending them in a buffer, or lyophilizing them.

The second antibody useful in the practice of this invention and specific to a particular immunoglobulin class is an antibody having three or more binding sites to the specific antibody to be assayed, i.e., a trivalent or higher polyvalent antibody (hereinafter referred to as "the polyvalent second antibody"). By using the polyvalent second antibody in this invention, a composite of the insoluble antigenic reagent and the specific antibody to be assayed is crosslinked through the polyvalent second antibody and hence polymerized. Therefore, the specific agglutination is amplified and the detection sensitivity is improved. No particular limitation is imposed on such a polyvalent second antibody so long as its valence is trivalent or higher. However, hexavalent or higher polyvalent antibodies, particularly, hexavalent to triacontavalent antibodies are preferred. Such polyvalent second antibodies may be either polyclonal or monoclonal. It is preferable to use antibodies obtained by artificially polymerizing an antibody of IgM or IgG type. The artificial polymerization of the antibody of IgG type is conducted by using a binder known per se in the art or the like. In the case where a biotin-avidin system (product of VECTOR LABORATORIES, INC.; agent: Funakoshi Yakuhin K.K.) is used by way of example, biotin is covalently bonded to an IgG type antibody. The biotin-bonded antibody thus obtained is reacted with a proper amount of avidin, whereby this antibody is polymerized into a polyvalent second antibody.

In the case where a polyclonal antibody is used, it is preferable to use that purified by affinity chromatography. A monoclonal antibody is prepared by immunizing a mouse against a human immunoglobulin of a particular class and then following the conventional method such as cell fusion.

The assay according to this invention is performed in accordance with the immunoassay making use of the conventional immunological agglutination except that the polyvalent second antibody is used as the second antibody. Namely, it is only necessary to add the diluted specimen into, for example, a liquid mixture of the insoluble antigenic reagent and the second antibody, incubate the mixture and then determine the degree of agglutination of the resulting aggregate.

It is only necessary to conduct the incubation usually for 10-30 minutes at 20-45°C. As the principle of detecting unaggregated particles and aggregated particles, a laser-scattering method, an electrical-resistance method (Coulter method) or the like may be used. In order to measure the degree of agglutination, it is only necessary to determine the amount of particles having a particle size corresponding to that of the insoluble antigenic reagent and the amount of particles having a particle size corresponding to that of the aggregated particles formed by aggregation of the insoluble antigenic reagent. More specifically, for example, a ratio (rate of reaction) of the amount of aggregated particles to the amount of unaggregated particles or the like is determined by using a sample of an antibody to be assayed, the concentration of which has been known, to prepare a calibration curve in advance. The amount of a specific antibody to be assayed in a sample specimen can be determined on the basis of this calibration curve. Incidentally, in the case where the method of this invention is performed by a particle meter according to the conventional electrical-resistance method (Coulter principle), it is only necessary to adjust the orifice diameter of its aperture to 20-50 microns. Owing to the method of this invention, the amount of the specific antibody to be assayed can thus be determined exactly. As described above, the agglutination is amplified in this invention, so that the amount of aggregated particles can be determined in sharp distinction from unaggregated particles. Therefore, it is possible to assay the specific antibody without labelling the second antibody.

According to the present invention, a trivalent or higher polyvalent second antibody is used as a second antibody. Therefore, a composite of the insoluble antigenic reagent and the specific antibody to be assayed is crosslinked through the polyvalent second antibody and hence polymerized, so that the agglutination is amplified. As a result, the detection sensitivity is sharply improved. It has accordingly been possible to exactly determine the amount of the specific antibody in the particular class. Further, the assay of this invention is not only good in sensitivity, but also simple in operation. The assaying time is also as short as at most 1 hour from blood-collecting, and the assay is thus completed promptly. The method of this invention is therefore extremely useful in medical treatments, clinical examinations, etc.

## EXAMPLES

The present invention will hereinafter be described more specifically by the following examples. However, it should be borne in mind that this invention is not limited to and by these examples.

### Example 1: Preparation of mite allergen-sensitized latex reagent

To 10 ml of a 1.0% (w/v) suspension of a polystyrene latex having a particle size of 1.0 micron in a 50 mM phosphate buffer containing 0.01% (w/v) of a surfactant "Triton X-100" (pH: 7.0, hereinafter referred to as "the preblocking buffer"), was added 0.25 ml of a solution with 100 mM of disuccinimidyl glutarate (BISHOX) dissolved in dimethylsulfoxide (DMSO). The liquid mixture was stirred and then held for 5 minutes at 37°C, followed by centrifugation to remove excess BISHOX by washing. The residue was resuspended in 10 ml of the preblocking buffer. The thus-obtained suspension was added with 10 ml of a 1.0 mg/ml solution of a mite allergen in a 0.1 M sodium carbonate buffer containing 0.15 M of sodium chloride (pH: 8.3, hereinafter referred to as "the coupling buffer"). The resulting mixture was thoroughly stirred to incubate it for 30 minutes at 4°C. The mixture was centrifuged to remove the residual allergen solution and buffer by washing. The residue was finally resuspended in 50 ml of a 50 mM HEPES buffer containing 10.0% (w/v) of sucrose, 0.2% (w/v) of bovine serum albumin (BSA) and 0.1% (w/v) of sodium azide (pH: 6.8, hereinafter referred to as "the final buffer") to preserve the suspension at 4°C.

### Example 2: Preparation of polyvalent second antibody making use of IgG antibody

Biotin was bonded to an anti-human IgG monoclonal antibody in accordance with a method known per se in the art [Hofmann, K. et al., J. Am. Chem. Soc., 100, 3585(1978)] to prepare a biotin-bonded anti-human IgG antibody with which streptoavidin in an amount 4 to 15 times in terms of molecular weight ratio was then reacted. The polymerized antibody was then separated by liquid chromatography, thereby preparing anti-human IgG polyvalent second antibodies having average antibody valences of 8 (corresponding to 4 molecules) and 30 (corresponding to 15 molecules), respectively. They were dialyzed with the final buffer to preserve them at 4°C.

Example 3: Difference in assaying sensitivity (amplifying effect) according to the kind of second antibody in mite-specific IgG antibody assay

Portions of a liquid reaction mixture obtained by reacting 50 microliters of a sample diluted with a 50 mM HEPES buffer containing 5.0% (w/v) of sucrose, 1.0% (w/v) of BSA, 0.5%(w/v) of sodium chloride and 0.1% (w/v) of sodium azide (pH: 6.8, hereinafter referred to as "the dilution buffer") with 25 microliters of the mite allergen-sensitized latex reagent prepared in Example 1 were mixed separately with the final buffer alone, a 0.1 mg/ml solution of an IgG type anti-human IgG antibody in the final buffer, the 0.02 mg/ml anti-human IgG polyvalent antibody solutions prepared in Example 2 and having antibody valences of 8 and 30, respectively, and a 0.02 mg/ml solution of an IgM type anti-human IgG monoclonal antibody in the final buffer, all, in amounts of 25 microliters. Each of the resulting mixture was incubated for 30 minutes at 37°C. After 5.0 ml of a 50 mM Bis-Tris buffer containing 0.1% (w/v) of sodium azide (pH: 6.3, hereinafter referred to as "the stopper buffer") was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Dilution reaction curves as to the mite allergen specific IgG antibody in the sample, which had been drawn by plotting the dilution rate and RV on the axis of abscissas and the axis of ordinates, respectively, were shown in FIG. 1. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 4: Difference in assaying sensitivity (amplifying effect) according to the kind of second antibody in mite-specific IgA antibody assay

Portions of a liquid reaction mixture obtained by reacting 50 microliters of a sample diluted with the dilution buffer with 25 microliters of the mite allergen-sensitized latex reagent prepared in Example 1 were mixed separately with the final buffer alone, a 0.1 mg/ml solution of an IgG type anti-human IgA antibody in the final buffer, a 0.02 mg/ml anti-human IgA polyvalent (octavalent) antibody prepared in accordance with Example 2, and a 0.03 mg/ml solution of an IgM type (decavalent) anti-human IgA monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Dilution reaction curves as to the mite allergen-specific IgA antibody in the sample, which had been drawn by plotting the dilution rate and RV on the axis of abscissas and the axis of ordinates, respectively, were shown in FIG. 2. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 5: Preparation of cedar allergen-sensitized latex reagent

To 10 ml of a 1.0% (w/v) suspension of a polystyrene latex having a particle size of 1.0 micron in the preblocking buffer, was added 0.25 ml of a solution with 100 mM of disuccinimidyl glutarate (BISHOX) dissolved in dimethylsulfoxide (DMSO). The liquid mixture was stirred and then held for 5 minutes at 37°C, followed by centrifugation to remove excess BISHOX by washing. The residue was resuspended in 10 ml of the preblocking buffer. The thus-obtained suspension was added with 10 ml of a 2.0 mg/ml solution of a cedar allergen in the coupling buffer. The resulting mixture was thoroughly stirred to incubate it for 30 minutes at 4°C. The mixture was centrifuged to remove the residual allergen solution and buffer by washing. The residue was finally resuspended in 50 ml of the final buffer to preserve the suspension at 4°C.

Example 6: Difference in assaying sensitivity (amplifying effect) according to the kind of second antibody in cedar-specific IgG antibody assay

Portions of a liquid reaction mixture obtained by reacting 50 microliters of a sample diluted with the dilution buffer with 25 microliters of the cedar allergen-sensitized latex reagent prepared in Example 5 were mixed separately with the final buffer alone, a 0.1 mg/ml solution of an IgG type anti-human IgG antibody in the final buffer, the 0.02 mg/ml anti-human IgG polyvalent (octavalent) antibody prepared in Example 2, and a 0.02 mg/ml solution of an IgM type (decavalent) anti-human IgG monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Dilution reaction curves as to the cedar allergen-specific IgG antibody in the sample, which had been drawn by plotting the dilution rate and RV on the axis of abscissas and the axis of ordinates, respectively, were shown in FIG. 3. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 7: Preparation of rice allergen-sensitized latex reagent

To 10 ml of a 1.0% (w/v) suspension of a polystyrene latex having a particle size of 1.0 micron in the preblocking buffer, was added 0.25 ml of a solution with 100 mM of BISHOX dissolved in DMSO. The liquid mixture was stirred and then held for 5 minutes at 37°C, followed by centrifugation to remove excess BISHOX by washing. The residue was resuspended in 10 ml of the preblocking buffer. The thus-obtained suspension was added with 10 ml of a 2.0 mg/ml solution of a rice allergen in the coupling buffer. The resulting mixture was thoroughly stirred to incubate it for 30 minutes at 4°C. The mixture was centrifuged to remove the residual allergen solution and buffer by washing. The residue was finally resuspended in 50 ml of the final buffer to preserve the suspension at 4°C.

Example 8: Difference in assaying sensitivity (amplifying effect) according to the kind of second antibody in cedar-specific IgG antibody assay

Portions of a liquid reaction mixture obtained by reacting 50 microliters of a sample diluted with the dilution buffer with 25 microliters of the rice allergen-sensitized latex reagent prepared in Example 7 were mixed separately with the final buffer alone, a 0.1 mg/ml solution of an IgG type anti-human IgG antibody in the final buffer, the 0.02 mg/ml anti-human IgG polyvalent (octavalent) antibody prepared in Example 2, and a 0.02 mg/ml solution of an IgM type (decavalent) anti-human IgG monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-tenth. The degree of agglutination of the thus-diluted reaction aggregate was determined in terms of change in turbidity (absorbance) at a wavelength of 650 nm.

Dilution reaction curves as to the rice allergen-specific IgG antibody in the sample, which had been drawn by plotting the dilution rate and the absorbance on the axis of abscissas and the axis of ordinates, respectively, were shown in FIG. 4. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 9: Difference in assaying sensitivity (amplifying effect) according to the kind of second antibody in rice-specific IgA antibody assay

Portions of a liquid reaction mixture obtained by reacting 50 microliters of a sample diluted with the dilution buffer with 25 microliters of the rice allergen-sensitized latex reagent prepared in Example 7 were mixed separately with the final buffer alone, a 0.1 mg/ml solution of an IgG type anti-human IgA antibody in the final buffer, a 0.02 mg/ml anti-human IgA polyvalent (octavalent) antibody prepared in accordance with Example 2, and a 0.02 mg/ml solution of an IgM type (decavalent) anti-human IgG monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-tenth. The degree of agglutination of the thus-diluted reaction aggregate was determined in terms of change in turbidity (absorbance) at a wavelength of 650 nm.

Dilution reaction curves as to the rice allergen-specific IgA antibody in the sample, which had been drawn by plotting the dilution rate and the absorbance on the axis of abscissas and the axis of ordinates, respectively, were shown in FIG. 5. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 10: Preparation of HBs (hepatitis B virus surface antigen)-sensitized latex reagent

To 10 ml of a 1.0% (w/v) suspension of a polystyrene latex having a particle size of 1.0 micron in the preblocking buffer, was added 0.25 ml of a solution with 100 mM of BISHOX dissolved in DMSO. The liquid mixture was stirred and then held for 5 minutes at 37°C, followed by centrifugation to remove excess BISHOX by washing. The residue was resuspended in 10 ml of the preblocking buffer. The thus-obtained suspension was added with 10 ml of a solution with 50 micrograms/ml of a BHs antigen dissolved in the coupling buffer. The resulting mixture was thoroughly stirred to incubate it for 30 minutes at 4°C. The mixture was centrifuged to remove the residual antigen solution and buffer by washing. The residue was finally resuspended in 50 ml of the final buffer to preserve the suspension at 4°C.

Example 11: Difference in assaying sensitivity (amplifying effect) according to the kind of second antibody in HBs-specific IgG antibody assay

Portions of a liquid reaction mixture obtained by reacting 50 microliters of a sample diluted with the dilution buffer with 25 microliters of the BHs-sensitized latex reagent prepared in Example 10 were mixed separately with the final buffer alone, a 0.1 mg/ml solution of an anti-human IgG antibody in the final buffer, the 0.02 mg/ml anti-human IgG polyvalent (octavalent) antibody prepared in Example 2, and a 0.02 mg/ml solution of an IgM type (decavalent) anti-human IgG monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Dilution reaction curves as to the HBs-specific IgG antibody in the sample, which had been drawn by plotting the dilution rate and RV on the axis of abscissas and the axis of ordinates, respectively, were shown in FIG. 6. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 12: Preparation of candida antigen-sensitized latex reagent

To 10 ml of a 1.0% (w/v) suspension of a polystyrene latex having a particle size of 1.0 micron in the preblocking buffer, was added 0.25 ml of a solution with 100 mM of BISHOX dissolved in DMSO. The liquid mixture was stirred and then held for 5 minutes at 37°C, followed by centrifugation to remove excess BISHOX by washing. The residue was resuspended in 10 ml of the preblocking buffer. The thus-obtained suspension was added with 10 ml of a 1 mg/ml solution of a candida antigen in the coupling buffer. The resulting mixture was thoroughly stirred to incubate it for 30 minutes at 4°C. The mixture was centrifuged to remove the residual antigen solution and buffer by washing. The residue was finally resuspended in 50 ml of the final buffer to preserve the suspension at 4°C.

Example 13: Difference in assaying sensitivity (amplifying effect) according to the kind of second antibody in candida-specific IgG antibody assay

Portions of a liquid reaction mixture obtained by reacting 50 microliters of a sample diluted with the dilution buffer with 25 microliters of the candida-sensitized latex reagent prepared in Example 12 were mixed separately with the final buffer alone, a 0.1 mg/ml solution of an anti-human IgG antibody in the final buffer, the 0.02 mg/ml anti-human IgG polyvalent (octavalent) antibody prepared in Example 2, and a 0.02 mg/ml solution of an IgM type (decavalent) anti-human IgG monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Dilution reaction curves as to the candida-specific IgG antibody in the sample, which had been drawn by plotting the dilution rate and RV on the axis of abscissas and the axis of ordinates, respectively, were shown in FIG. 7. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 14: Difference in assaying sensitivity (amplifying effect) according to the kind of second antibody in candida-specific IgM antibody assay

Portions of a liquid reaction mixture obtained by reacting 50 microliters of a sample diluted with the dilution buffer with 25 microliters of the candida-sensitized latex reagent prepared in Example 12 were mixed separately with the final buffer alone, a 0.1 mg/ml solution of an anti-human IgG antibody in the final buffer, a 0.01 mg/ml anti-human IgM polyvalent (octavalent) antibody prepared in accordance with Example 2, and a 0.01 mg/ml solution of an IgM type (decavalent) anti-human IgM monoclonal antibody in the final buffer, all, in amounts of 25 microliters. Each of the resulting mixture was incubated for 30 minutes at 37°C. After 5.0 ml of the stopper buffer was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Dilution reaction curves as to the candida-specific IgM antibody in the sample, which had been drawn by plotting the dilution rate and RV on the axis of abscissas and the axis of ordinates, respectively, were shown in FIG. 8. As a result, it was found that no reaction was recognized where any second antibody was not used, and the assaying sensitivity (amplifying effect on the agglutination) where the artificially polymerized second antibodies and IgM type second antibody (polyvalent second antibodies) were used was far higher than that of the IgG type (bivalent) second antibody.

Example 15: Confirmation of specificity to allergen in allergen-specific IgG antibody assay making use of second antibody (inhibition of reaction by addition of mite allergen in mite-specific IgG assay)

Twenty-five microliters of the dilution buffer alone or a sample diluted with the dilution buffer to one-hundredth, 25 microliters of a solution of a mite allergen in the dilution buffer or a solution of a human serum albumin (HSA) in the final buffer, 25 microliters of the mite allergen-sensitized latex reagent prepared in Example 1 and 25 microliters of a 0.02 mg/ml solution of an anti-human IgG monoclonal antibody in the final buffer were mixed with one another. Each of the resulting liquid mixtures was incubated for 30 minutes at 37°C. After 3.0 ml of the stopper buffer was then added to stop the reaction, the reaction mixture was diluted further with an electrolyte to one-thirtieth. The particle size distribution of the thus-diluted reaction aggregate was determined by a particle meter ("PCIA meter", trade name) to calculate the degree of agglutination (RV: a ratio of the total volumes of unaggregated latex and aggregated latex).

Reaction curves drawn by plotting the concentration of the added mite allergen or HSA, and RV on the axis of abscissas and the axis of ordinates, respectively, were shown in FIG. 9. As shown in the drawing, the inhibition by HSA was not recognized. With respect to the mite allergen, inhibition became recognized as its concentration added increased.

## Claims

1. An assay of a specific antibody to an antigen in a particular immunoglobulin class and/or subclass by an immunoassay wherein an immunological agglutination making use of antigen-sensitized insoluble carrier particles and a second antibody specific to the particular immunoglobulin class and/or subclass is used, which comprises using a trivalent or higher polyvalent antibody specific to the particular immunoglobulin class and/or subclass as the second antibody.

2. The assay according to claim 1, wherein the second antibody is an antibody obtained by artificially polymerizing an IgG type monoclonal antibody.

3. The assay according to claim 1, wherein the second antibody is an IgM type monoclonal antibody.

4. The assay according to claim 1, wherein the second antibody is an antibody obtained by artificially polymerizing an affinity-purified polyclonal antibody.

FIG. 1

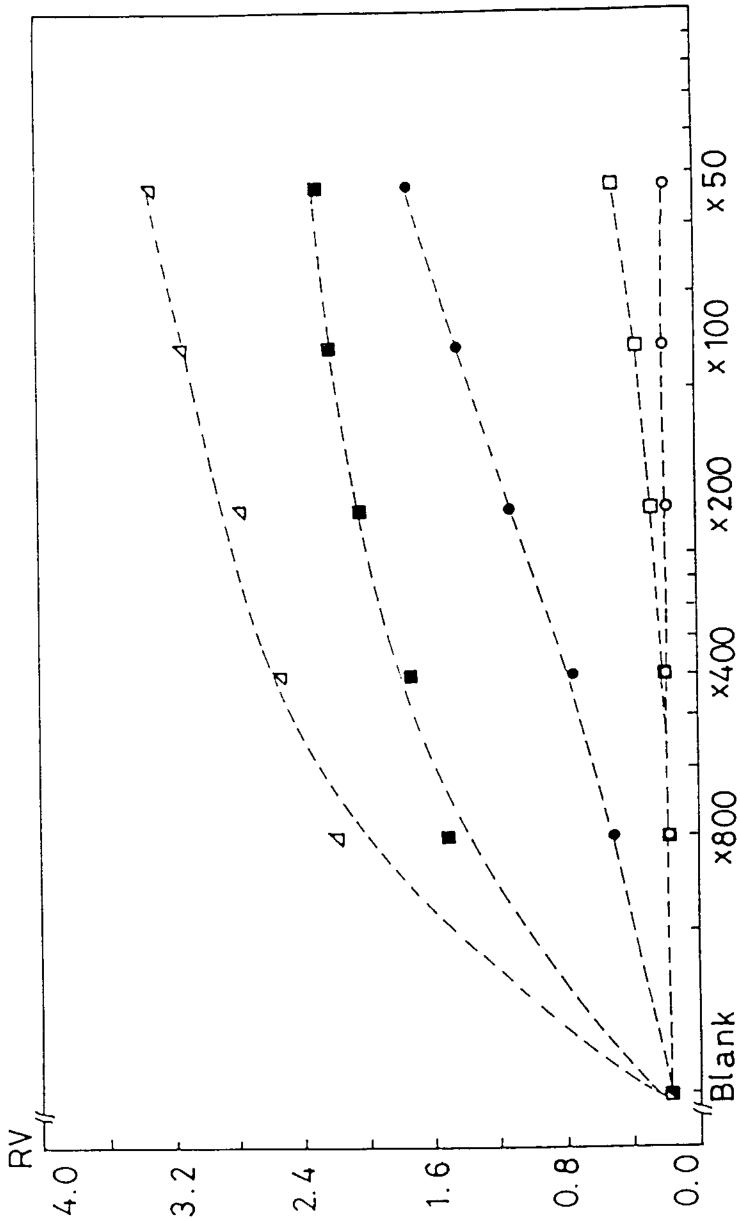


o: Any second antibody was not used.

□: IgG type (bivalent) second antibody was used.

●: Artificial polyvalent (octavalent) second antibody was used.

■: IgM type (decavalent) second antibody was used.

Δ: Artificial polyvalent (triacontavalent) second antibody was used.

FIG. 2

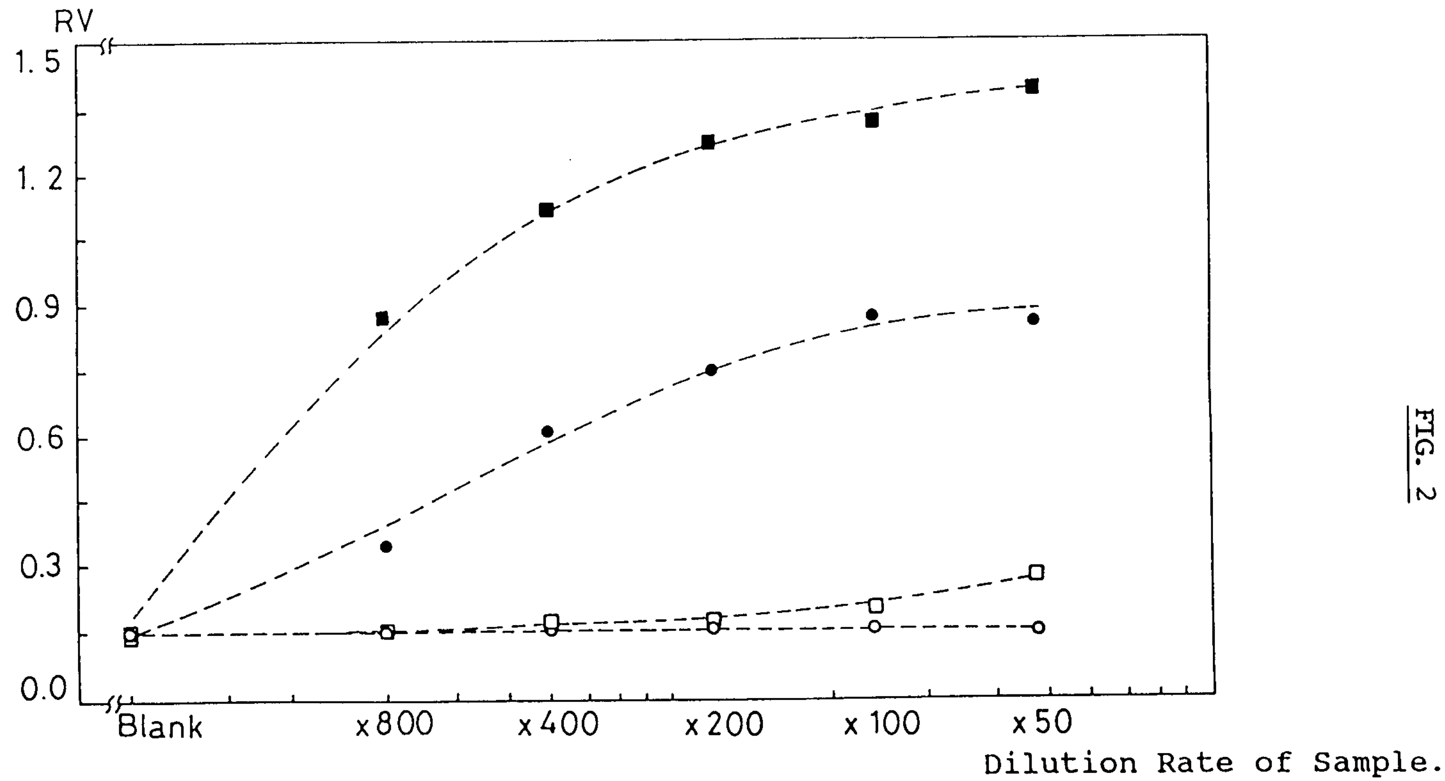


o: Any second antibody was not used.

□: IgG type (bivalent) second antibody was used.

●: Artificial polyvalent (octavalent) second antibody was used.

■: IgM type (decavalent) second antibody was used.

FIG. 3

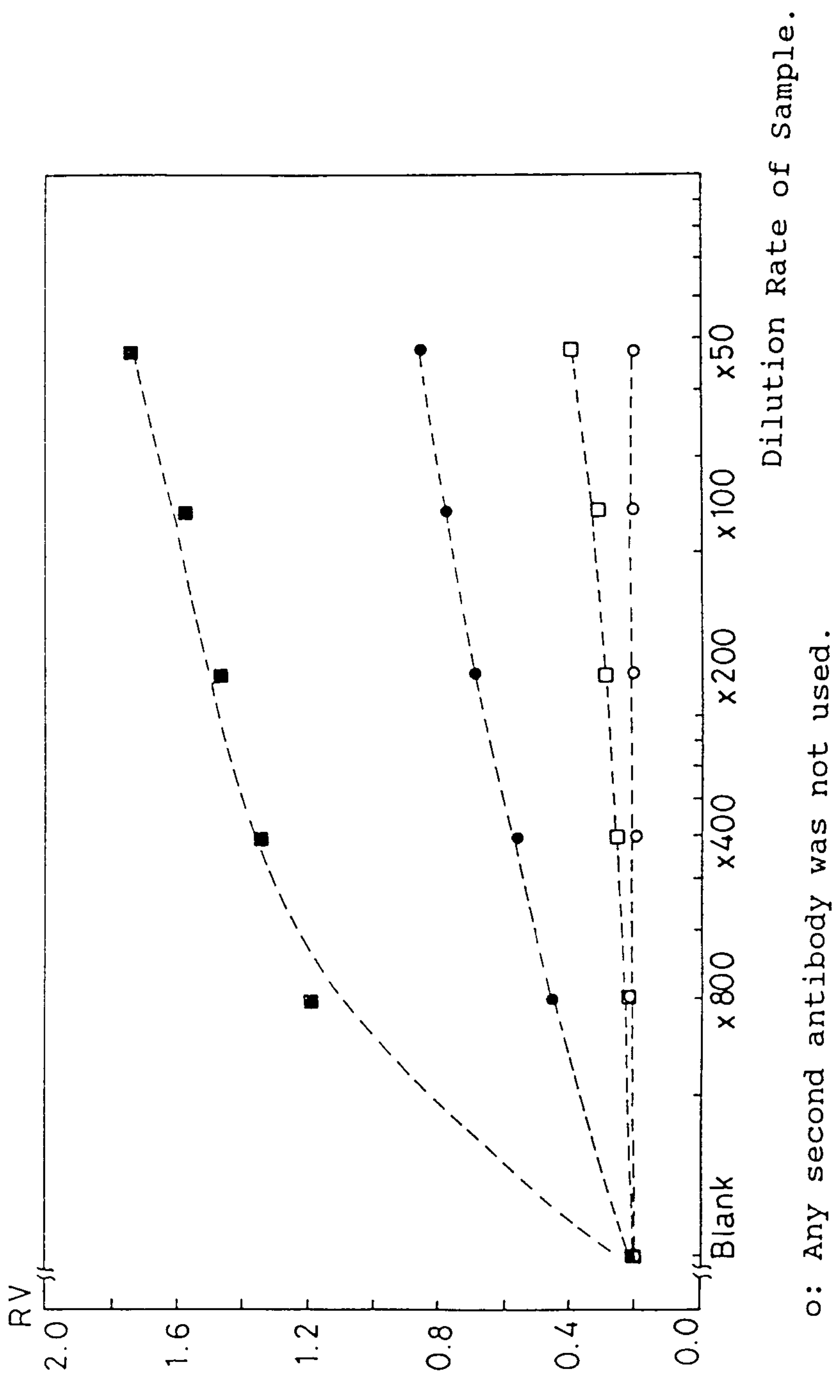


o: Any second antibody was not used.

□: IgG type (bivalent) second antibody was used.

●: Artificial polyvalent (octavalent) second antibody was used.

■: IgM type (decavalent) second antibody was used.

FIG. 4

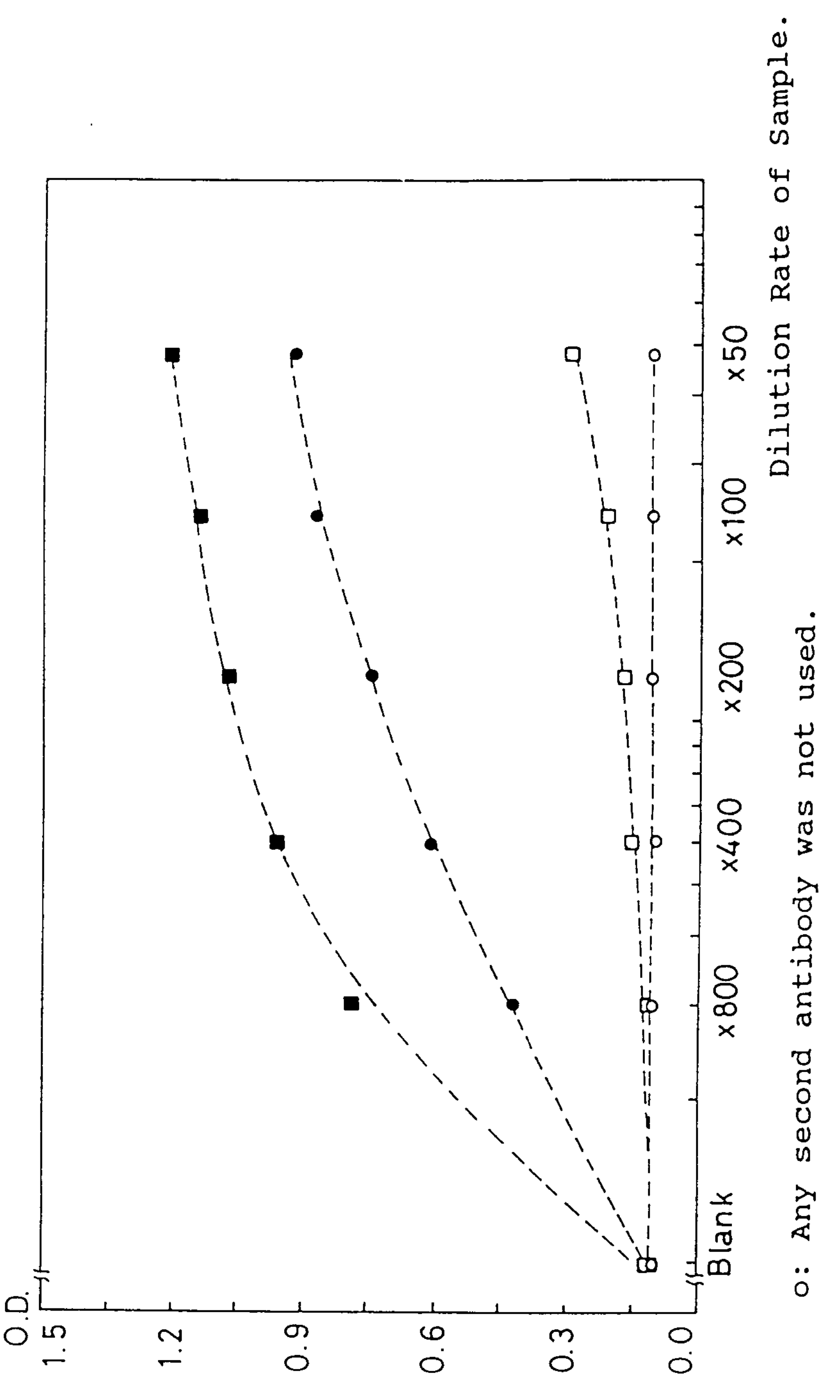


o: Any second antibody was not used.

□: IgG type (bivalent) second antibody was used.

●: Artificial polyvalent (octavalent) second antibody was used.

■: IgM type (decavalent) second antibody was used.

FIG. 5

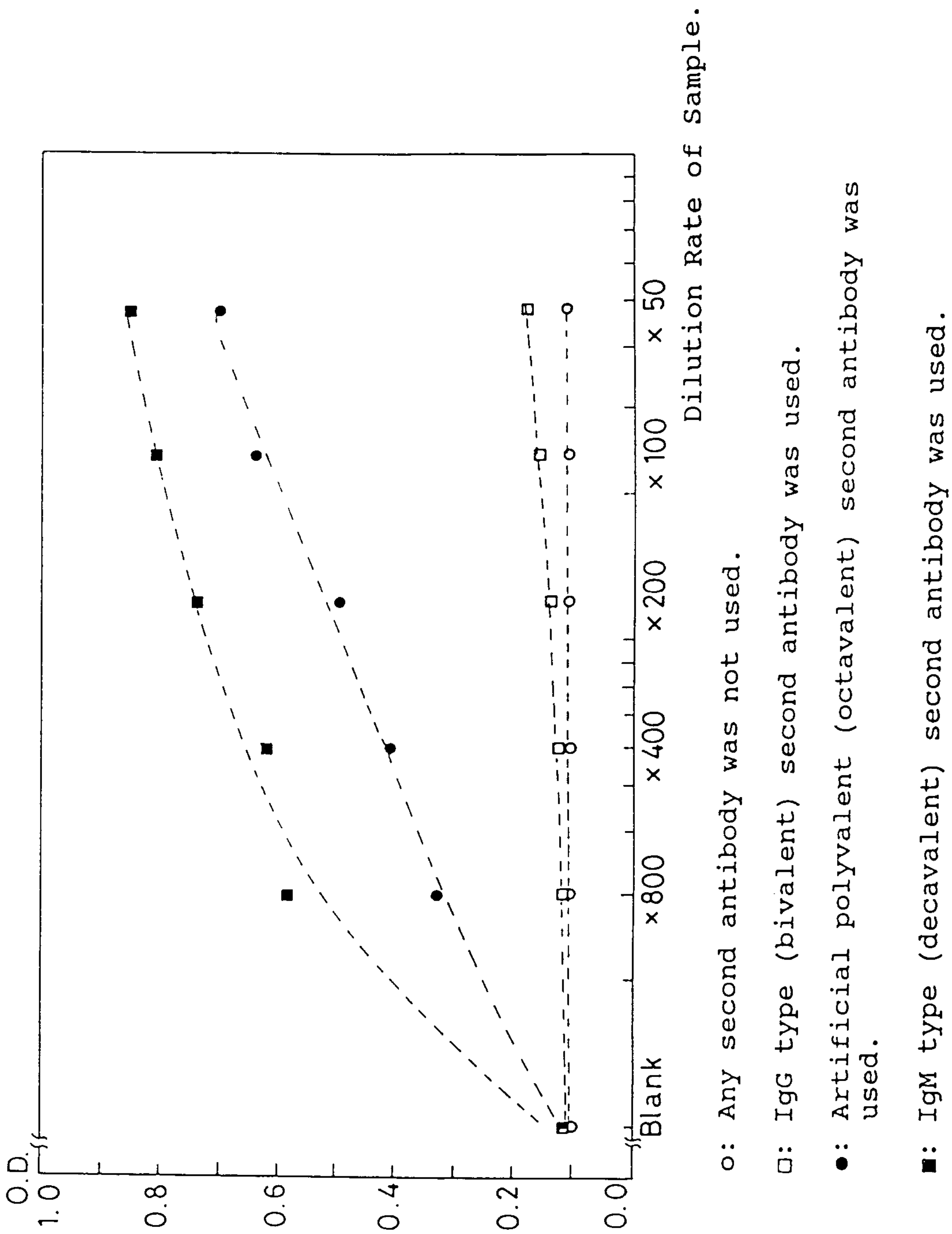
O.D.
1.0
0.8
0.6
0.4
0.2
0.0
Blank
×800
×400
×200
×100
×50
Dilution Rate of Sample.
o: Any second antibody was not used.
□: IgG type (bivalent) second antibody was used.
●: Artificial polyvalent (octavalent) second antibody was used.
■: IgM type (decavalent) second antibody was used.

FIG. 6

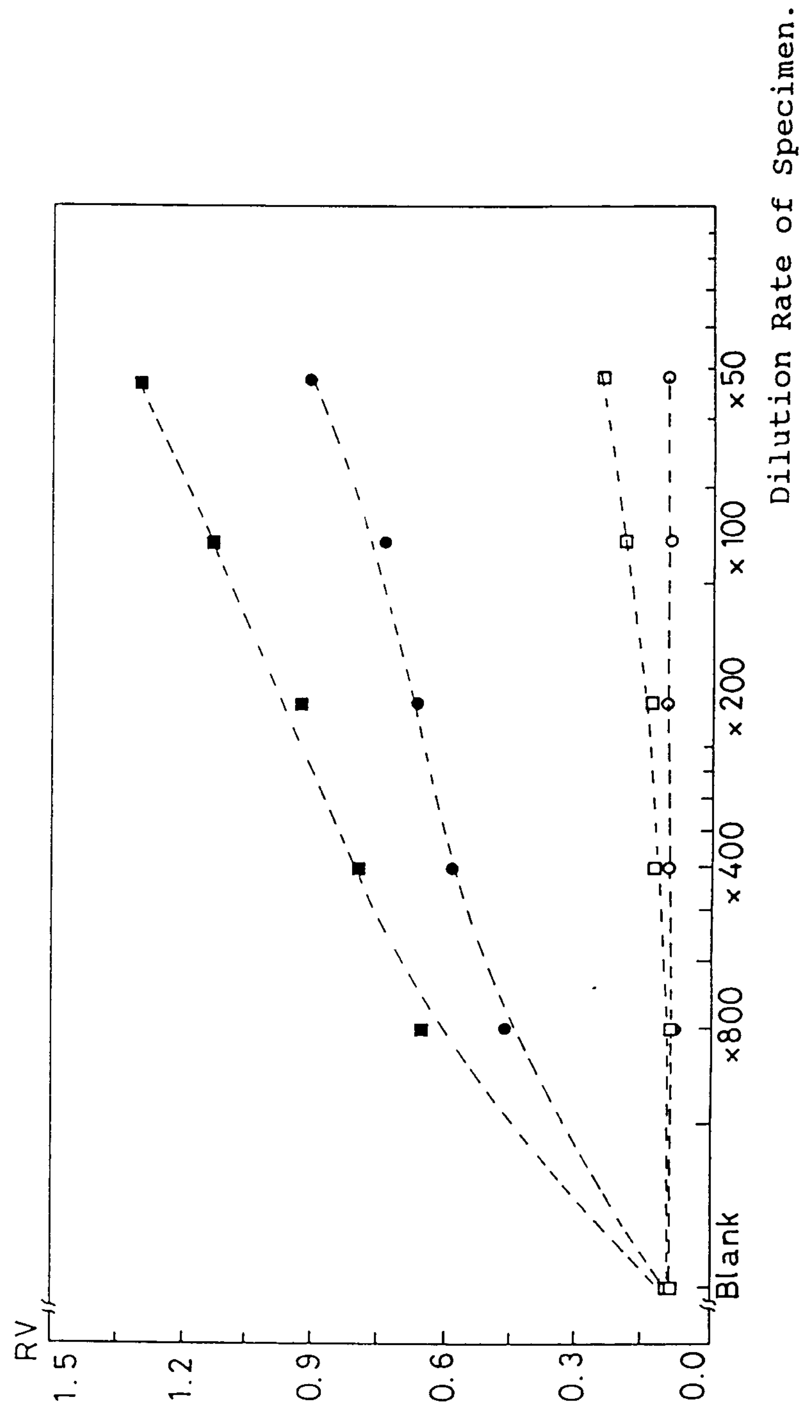


o: Any second antibody was not used.

□: IgG type (bivalent) second antibody was used.

●: Artificial polyvalent (octavalent) second antibody was used.

■: IgM type (decavalent) second antibody was used.

FIG. 7

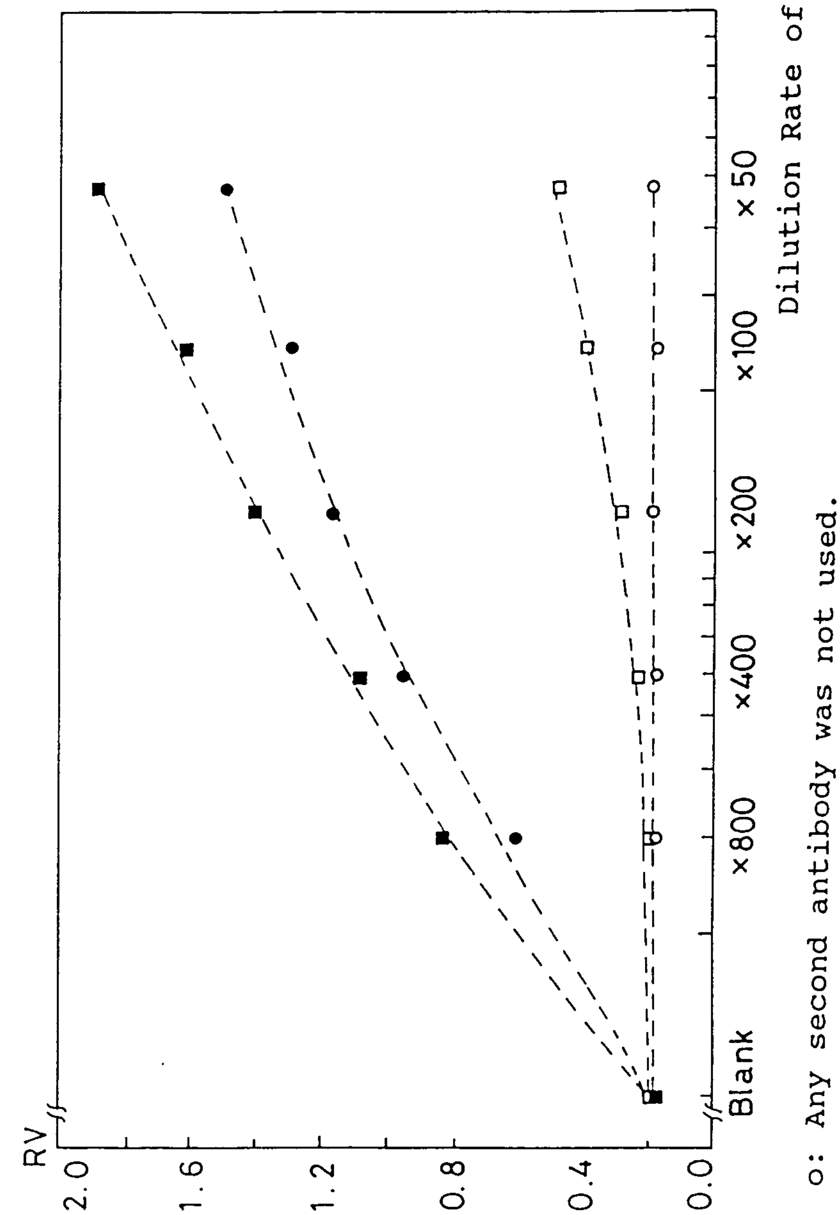


○: Any second antibody was not used.

□: IgG type (bivalent) second antibody was used.

●: Artificial polyvalent (octavalent) second antibody was used.

■: IgM type (decavalent) second antibody was used.

FIG. 8

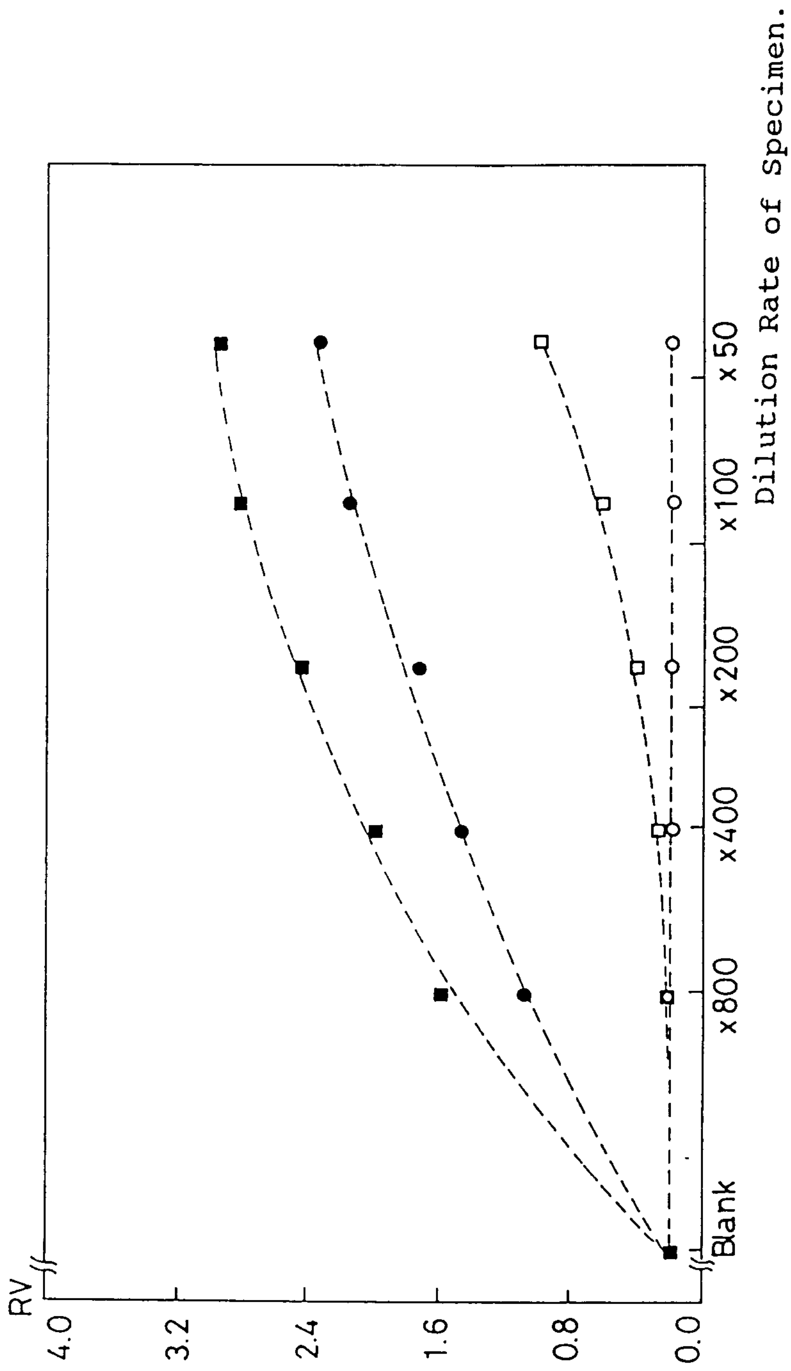


o: Any second antibody was not used.

□: IgG type (bivalent) second antibody was used.

●: Artificial polyvalent (octavalent) second antibody was used.

■: IgM type (decavalent) second antibody was used.

FIG. 9

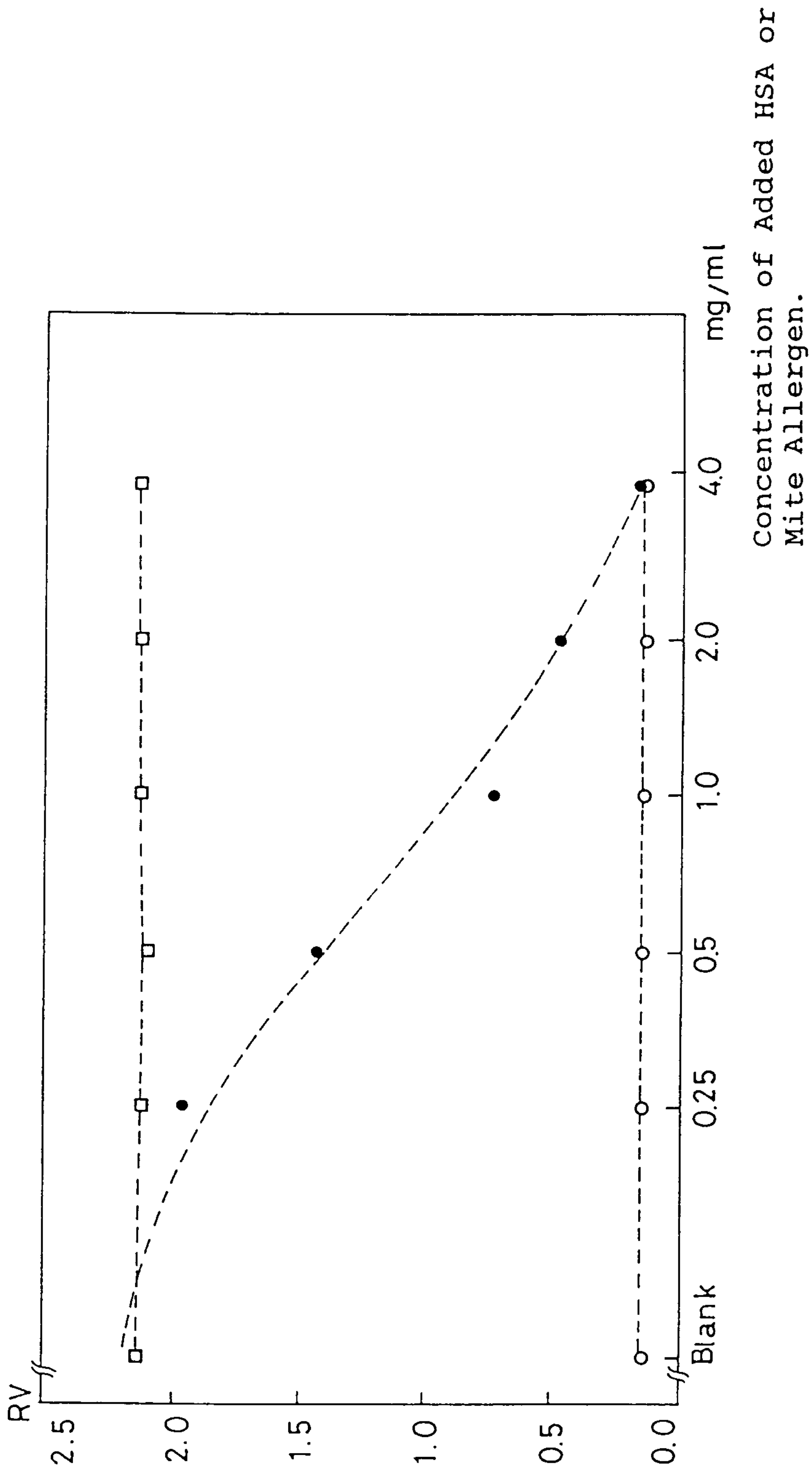


o: HSA was added to the sample diluted to one-hundredth.

□: Mite allergen was added to the sample diluted to one-hundredth.

●: Mite allergen was added to a blank.